(19) Europäisches Patentamt
European Patent Office
Office européen des brevets

(11) Veröffentlichungsnummer: **0 487 996 A2**

(12) **EUROPÄISCHE PATENTANMELDUNG**

(21) Anmeldenummer: **91119526.1**

(22) Anmeldetag: **15.11.91**

(51) Int. Cl.[5]: **C07D 501/20**, C07D 501/18, A61K 31/545, A23K 1/17

(30) Priorität: **28.11.90 DE 4037841**

(43) Veröffentlichungstag der Anmeldung: **03.06.92 Patentblatt 92/23**

(84) Benannte Vertragsstaaten: **AT BE CH DE DK ES FR GB GR IT LI LU NL SE**

(71) Anmelder: **BAYER AG**
**W-5090 Leverkusen 1 Bayerwerk(DE)**

(72) Erfinder: **Schneider, Stephan, Dr.**
**Claudiusweg 3**
**W-5600 Wuppertal 1(DE)**
Erfinder: **Endermann, Rainer, Dr.**
**In den Birken 152a**
**W-5600 Wuppertal 1(DE)**
Erfinder: **Metzger, Karl Georg, Dr.**
**Pahlkestrasse 75**
**W-5600 Wuppertal 1(DE)**
Erfinder: **Bremm, Klaus-Dieter, Dr.**
**Vogelsauer 59**
**W-5600 Wuppertal(DE)**

(54) **Neue 3-substituierte Cephalosporine, Verfahren zu ihrer Herstellung und ihre Verwendung als Arzneimittel.**

(57) Die Erfindung betrifft neue 3-substituierte Cephalosporine, Verfahren zu ihrer Herstellung und ihre Verwendung als Arzneimittel, insbesondere als antibakteriell wirksame Arzneimittel.

Die Erfindung betrifft Verbindungen der allgemeinen Formel (I),

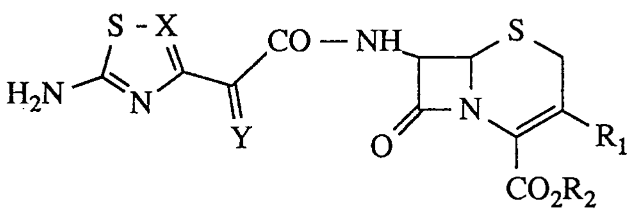

(I)

in welcher

X, Y, $R^1$ und $R^2$ die in der Beschreibung angegebene Bedeutung haben.

EP 0 487 996 A2

Die Erfindung betrifft neue 3-substituierte Cephalosporine, Verfahren zu ihrer Herstellung und ihre Verwendung als Arzneimittel, insbesondere als antibakteriell wirksame Arzneimittel.

Aus der EP 192 210 A2 sind antibakteriell wirksame 7-substituierte-Cyclopropyl-oximino-acetamido-cephem-carbonsäuren bekannt.

Außerdem werden in der EP A2 223 184 7-Thiazolyl-8-butenoylamino-cephalosporinderivate beschrieben.

Die Erfindung betrifft Verbindungen der allgemeinen Formel (I),

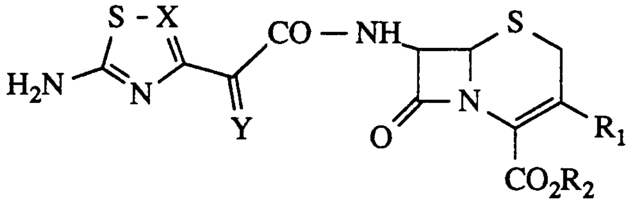

(I)

in welcher

| | |
|---|---|
| X | für ein Stickstoffatom oder für die -CH-Gruppe steht, |
| Y | für eine Gruppe der Formel N-$OR^3$ oder $CHR^4$ steht, worin |
| $R^3$ | Wasserstoff, geradkettiges oder verzweigtes Alkenyl, Alkinyl oder Alkyl mit jeweils bis zu 8 Kohlenstoffatomen bedeutet, wobei letzteres gegebenenfalls durch Halogen oder durch geschütztes oder ungeschütztes Carboxy oder Amino substituiert sein kann, |
| $R^4$ | Wasserstoff, Aryl mit 6 bis 10 Kohlenstoffatomen, geschütztes oder ungeschütztes Carboxy, Halogen oder geradkettiges oder verzweigtes Alkoxycarbonyl, Alkoxy, Alkenyl oder Alkyl mit jeweils bis zu 8 Kohlenstoffatomen bedeutet, wobei letzteres gegebenenfalls durch Halogen, Hydroxy, Nitro, Cyano, Carboxy oder durch geradkettiges oder verzweigtes Alkoxy oder Alkoxycarbonyl mit jeweils bis zu 6 Kohlenstoffatomen substituiert sein kann, |
| $R^1$ | für einen Rest der Formel |

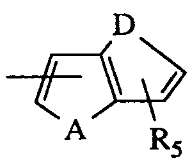

oder

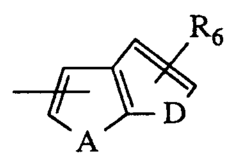

steht,
worin

| | |
|---|---|
| A und D | gleich oder verschieden sind und die Gruppe -CH=CH oder ein Sauerstoff- oder Schwefelatom bedeuten und |
| $R^5$ und $R^6$ | gleich oder verschieden sind und Halogen, Trifluormethyl, Trifluormethoxy, Hydroxy oder eine Gruppe der Formel -$NR^7R^8$ bedeuten, worin |
| $R^7$ oder $R^8$ | gleich oder verschieden sind und Wasserstoff, geradkettiges oder verzweigtes Alkyl mit bis zu 8 Kohlenstoffatomen oder Phenyl bedeuten, oder geradkettiges oder verzweigtes Alkoxy oder Alkyl mit jeweils bis zu 8 Kohlenstoffatomen bedeuten, wobei letzteres durch Hydroxy, Halogen oder durch geradkettiges oder verzweigtes Alkoxy mit bis zu 6 Kohlenstoffatomen substituiert sein kann, |
| $R^2$ | für Wasserstoff, oder für eine in der β-Lactamchemie übliche Carboxyschutzgruppe oder für einen in vivo spaltbaren Esterrest steht |

und deren pharmazeutisch verträglichen Salze.

Wegen der Anwesenheit der Doppelbindungen (=Y) können die erfindungsgemäßen Verbindungen der allgemeinen Formel (I) als reine syn- oder anti-Isomere oder als Gemische von Isomeren auftreten.

Bevorzugt sind die syn-Isomere der erfindungsgemäßen Verbindungen der allgemeinen Formel (I).

Sowohl die Isomerengemische als auch die syn- und anti-Form der erfindungsgemäßen Verbindungen können zur Behandlung bakterieller Infektionskrankheiten eingesetzt werden.

Die Verbindungen der allgemeinen Formel (I) können als freie Säuren, Ester, als innere Salze oder als nicht-toxische pharmazeutisch verträgliche Salze der sauren Carboxylgruppen wie Natrium-, Kalium-, Magnesium-, Calcium-, Aluminium- oder Ammoniumsalze, mit Aminen wie Di-, Tri-niedrigalkylaminen, Procain, Dibenzylamin, N,N'-Dibenzylethylendiamin, N-Benzyl-β-phenyl-ethylamin, N-Methyl- und N-Ethylmorpholin, 1-Ephenamin, Dihydroabiethylamin, N,N'-Bis-dehydroabiethylethylendiamin, N-Niedrigalkylpiperidin und anderen Aminen, die zur Bildung von Salzen von Penicillinen und Cephalosporinen verwendet werden können, vorliegen.

Als nicht-toxische, pharmazeutisch verträgliche Salze der basischen Aminogruppen mit anorganischen oder organischen Säureresten sind beispielsweise Chlorid, Bromid, Iodid, Sulfat, Hydrogensulfat, Phosphat, Carbonat, Hydrogencarbonat, oder Sulfonate wie Methylsulfonat, Ethansulfonat, Toluolsulfonat, Benzolsulfonat, Naphthalindisulfonat, oder Carboxylate wie Acetat, Formiat, Oxalat, Tartrat, Citrat, Maleat, Fumarat, Benzoat, Succinat oder Lactat bevorzugt zu nennen.

Amino- oder Oximschutzgruppe im Rahmen der oben angegebenen Definition steht im allgemeinen für eine in der β-Lactam-Chemie üblichen Schutzgruppe aus der Reihe: tert.Butoxycarbonyl, Benzyloxycarbonyl, 2-Nitrobenzyloxycarbonyl, 4-Nitrobenzyloxycarbonyl, Phenylacetyl, Allyloxycarbonyl, 2,4-Dimethoxybenzyloxycarbonyl, Allyloxymethyl, Bis-(4-methoxyphenyl)methyl, 2-(Methylthiomethoxy)ethoxycarbonyl, Trimethyl-, Triethyl-, Triphenylsilyl, Trityl, tert.Butyl-dimethylsilyl, tert.Butyldiphenylsilyl, [2-(Trimethylsilyl)-ethoxy]methyl, 1-Methyl-2-benzoyl-vinyl, 1-Methyl-2-methoxy-vinyl, 1-Methyl-2-acetyl-vinyl, 1-Methyl-2-(methoxybenzoyl)-vinyl, 1-Methyl-2-(2,6-dimethoxybenzoyl)vinyl, 1-Methyl-2-ethoxycarbonyl-vinyl.

Carboxyschutzgruppe im Rahmen der oben angegebenen Definition steht für die in der β-Lactam-Chemie üblichen Carboxyschutzgruppen. Bevorzugt sind leicht abspaltbare Gruppen zu nennen, wie zum Beispiel: tert.Butyl, 2,2,2-Trichlorethyl, Diphenylmethyl, Triphenylmethyl, Acetoxymethyl, Allyl, Benzyl, 4-Methoxyphenyl, 4-Nitrobenzyl, 2-Nitrobenzyl, 4-Methoxybenzyl, 2,4-Dimethoxybenzyl, Trimethylsilylethyl, Trimethylsilyl, tert.Butyl-dimethylsilyl, Acetonyl, 1-Phenoxyethyl oder 2-Methyl-2-propenyl.

Steht $R^2$ für einen in vivo leicht abspaltbaren Esterrest, so sind hiermit pharmazeutisch verträgliche Esterreste gemeint, die in vivo leicht zu freien Carboxylgruppen ($R^2$ = H) hydrolysiert werden.

Solche Esterreste sind auf dem β-Lactam-Gebiet gut bekannt. In den meisten Fällen bessern sie die Absorptionseigenschaften der β-Lactam-Verbindungen. Außerdem sollte der Rest $R^2$ von solcher Art sein, daß er einer Verbindung der Formel (I) pharmazeutisch annehmbare Eigenschaften verleiht und bei Spaltung in vivo pharmazeutisch annehmbare Fragmente freisetzt.

Beispiele für solche Gruppen befinden sich in der DE-OS 2 517 316. Bevorzugte in vivo abspaltbare Estergruppen sind die der folgenden Formeln:

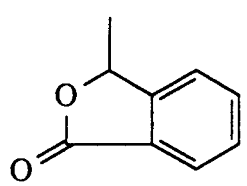 , 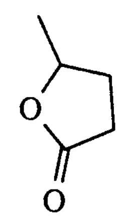 , 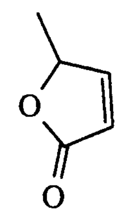 , $-CH(CO_2C_2H_5)-OC_2H_5$

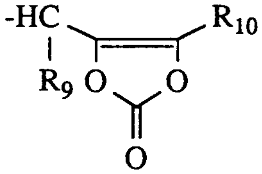 , 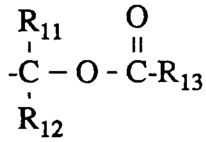 oder 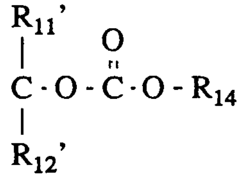

worin

| | |
|---|---|
| $R^9$ und $R^{10}$ | gleich oder verschieden sind und Wasserstoff, Phenyl oder geradkettiges oder verzweigtes Alkyl mit bis zu 4 Kohlenstoffatomen bedeuten, |
| $R^{11}$, $R^{11'}$, $R^{12}$ und $R^{12'}$ | gleich oder verschieden sind und |

$R^{11}$, $R^{11'}$, $R^{12}$ und $R^{12'}$ gleich oder verschieden sind und Wasserstoff oder geradkettiges oder verzweigtes Alkyl mit bis zu 4 Kohlenstoffatomen bedeuten,

$R^{13}$ geradkettiges oder verzweigtes Alkyl mit bis zu 6 Kohlenstoffatomen bedeutet,

$R^{14}$ geradkettiges oder verzweigtes Alkyl mit bis zu 6 Kohlenstoffatomen oder Cyclohexyl bedeutet.

Bevorzugt sind Verbindungen der allgemeinen Formel (I), in welcher

X für ein Stickstoffatom oder für die -CH-Gruppe steht,

Y für eine Gruppe der Formel N-$OR^3$ oder CH-$R^4$ steht, worin

$R^3$ Wasserstoff oder geradkettiges oder verzweigtes Alkyl mit bis zu 6 Kohlenstoffatomen bedeutet, das gegebenenfalls durch Fluor, Chlor, Brom oder geschütztes oder ungeschütztes Carboxy oder Amino substituiert ist,

$R^4$ Wasserstoff, Phenyl, Carboxy, Fluor, Chlor, Brom oder geradkettiges oder verzweigtes Alkoxy, Alkoxycarbonyl oder Alkyl mit jeweils bis zu 6 Kohlenstoffatomen bedeutet, wobei letzteres durch Fluor, Chlor, Brom, Hydroxy, Carboxy oder durch geradkettiges oder verzweigtes Alkoxy oder Alkoxycarbonyl mit jeweils bis zu 4 Kohlenstoffatomen substituiert sein kann,

$R^1$ für einen Rest der Formel

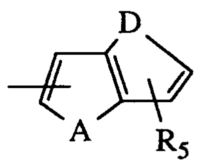

oder

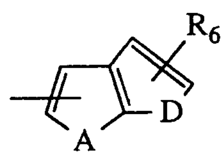

steht worin

A ein Schwefelatom bedeutet und

D die -CH = CH-Gruppe oder ein Schwefelatom bedeutet,

$R^5$ und $R^6$ gleich oder verschieden sind und Fluor, Chlor, Brom, Amino oder geradkettiges oder verzweigtes Alkoxy oder Alkyl mit jeweils bis zu 4 Kohlenstoffatomen bedeuten, wobei letzteres durch Hydroxy, Fluor, Chlor, Methoxy oder Ethoxy substituiert sein kann,

$R^2$ für Wasserstoff steht, oder für einen Rest der Formel

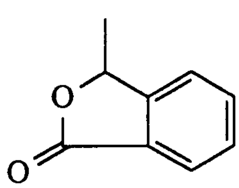

,

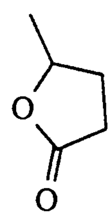

,

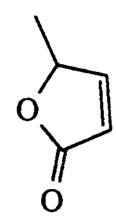

,

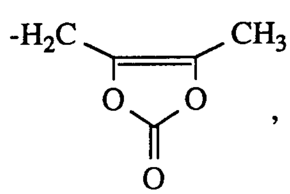

,

$-CH_2-OCO-C(CH_3)_3$ ,

$-CH(CH_3)-OCOOC_2H_5$ ,
$-CH_2-OCOCH_3$ ,
$-CH-(CH_3)-O-CO-CH_3$ ,
$-CH(CH_3)-O-COO-CH_3$ , $-CH(CH_3)-O-COO-CH(CH_3)_2$ ,
$-CH(CH_3)-O-COO-C_6H_{11}$ oder $-CH(CO_2C_2H_5)-OC_2H_5$ steht

und deren pharmazeutische verträgliche Salze.

Besonders bevorzugt sind Verbindungen der allgemeinen Formel (I)
in welcher

X für ein Stickstoffatom oder für die -CH-Gruppe steht,

Y für eine Gruppe der Formel $N-OR^3$ oder $-CHR^4$ steht,
worin

$R^3$ Wasserstoff oder geradkettiges oder verzweigtes Alkyl mit bis zu 4 Kohlenstoffatomen bedeutet, das gegebenenfalls durch Fluor, Chlor oder durch geschütztes oder ungeschütztes Carboxy oder Amino substituiert ist,

$R^4$ Wasserstoff oder geradkettiges oder verzweigtes Alkoxy oder Alkyl mit jeweils bis zu 4 Kohlenstoffatomen bedeutet, wobei letzteres durch Hydroxy, Carboxy, Methoxy, Ethoxy oder Propoxy substituiert sein kann,

$R^1$ für einen Rest der Formel

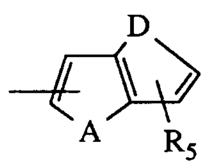

oder

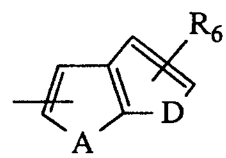

steht
worin

A ein Schwefelatom bedeutet
und

D die -CH = CH-Gruppe oder ein Schwefelatom bedeutet,

$R^5$ und $R^6$ gleich oder verschieden sind und Fluor, Chlor, Brom, Amino oder geradkettiges oder verzweigtes Alkoxy oder Alkyl mit jeweils bis zu 4 Kohlenstoffatomen bedeuten, wobei letzteres durch Hydroxy, Fluor, Chlor, Methoxy oder Ethoxy substituiert sein kann,

$R^2$ für Wasserstoff steht, oder
für einen Rest der Formel

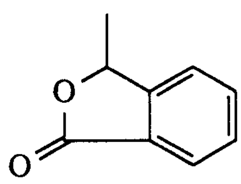 ,

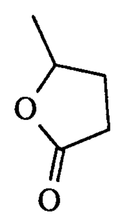 ,

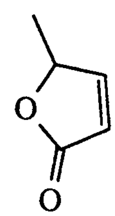 ,

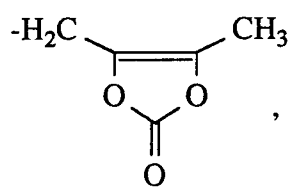 ,

$-CH_2-OCO-C(CH_3)_3$ ,
$-CH(CH_3)-OCOOC_2H_5$ ,
$-CH_2-OCOCH_3$ ,

$-CH-(CH_3)-O-CO-CH_3$ ,
$-CH(CH_3)-O-COO-CH_3$ , $-CH(CH_3)-O-COO-CH(CH_3)_2$ ,
$-CH(CH_3)-O-COO-C_6H_{11}$ oder $-CH(CO_2C_2H_5)-OC_2H_5$ steht

und deren pharmazeutische verträgliche Salze.

Ganz besonders bevorzugt sind die Natriumsalz und die Hydrochloride.

Weiterhin wurde ein Verfahren zur Herstellung der erfindungsgemäßen Verbindungen der allgemeinen Formel (I) gefunden, dadurch gekennzeichnet, daß man Verbindungen der allgemeinen Formel (II),

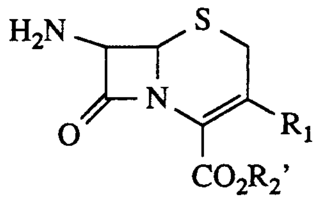

(II)

in welcher

$R^1$ die oben angegebene Bedeutung hat
und
$R^{2'}$ für eine der oben angegebenen Carboxyschutzgruppen, insbesondere für p-Methoxybenzyl steht,

gegebenenfalls auch unter Aktivierung der Aminfunktion,
mit Verbindungen der allgemeinen Formel (III),

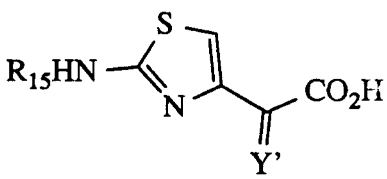

(III)

in welcher

Y' für eine der oben aufgeführten Gruppen N-$OR^{3'}$ oder $CHR^{4'}$ steht,

worin

$R^{3'}$ die oben angegebene Bedeutung von $R^3$ hat oder für eine der oben aufgeführten Oximschutzgruppen, insbesondere für den Tritylrest steht,

$R^{4'}$ die oben angegebene Bedeutung von $R^4$ hat oder für geschütztes Carboxy steht

und

$R^{15}$ ebenfalls für eine der oben aufgeführten Aminoschutzgruppen, vorzugsweise für Boc oder für den Tritylrest steht,

in inerten Losemitteln, gegebenenfalls unter Aktivierung der Carboxylgruppe in Anwesenheit eines Hilfsstoffes,
zunächst zu den Verbindungen der allgemeinen Formel (IV),

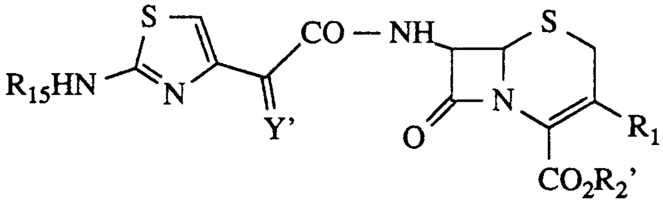

(IV)

in welcher

$R^1$, $R^{2'}$, $R^{15}$ und Y' die oben angegebene Bedeutung haben,

umsetzt und anschließend die Schutzgruppe $R^{2'}$, $R^{3'}$, $R^{4'}$ (Y') und $R^{15}$ nach üblicher Methode, gegebenenfalls in 2 Schritten abspaltet und die gewünschten Salze oder aus den Salzen die freien Säuren herstellt.

Das erfindungsgemäße Verfahren kann durch folgendes Formelschema beispielhaft erläutert werden:

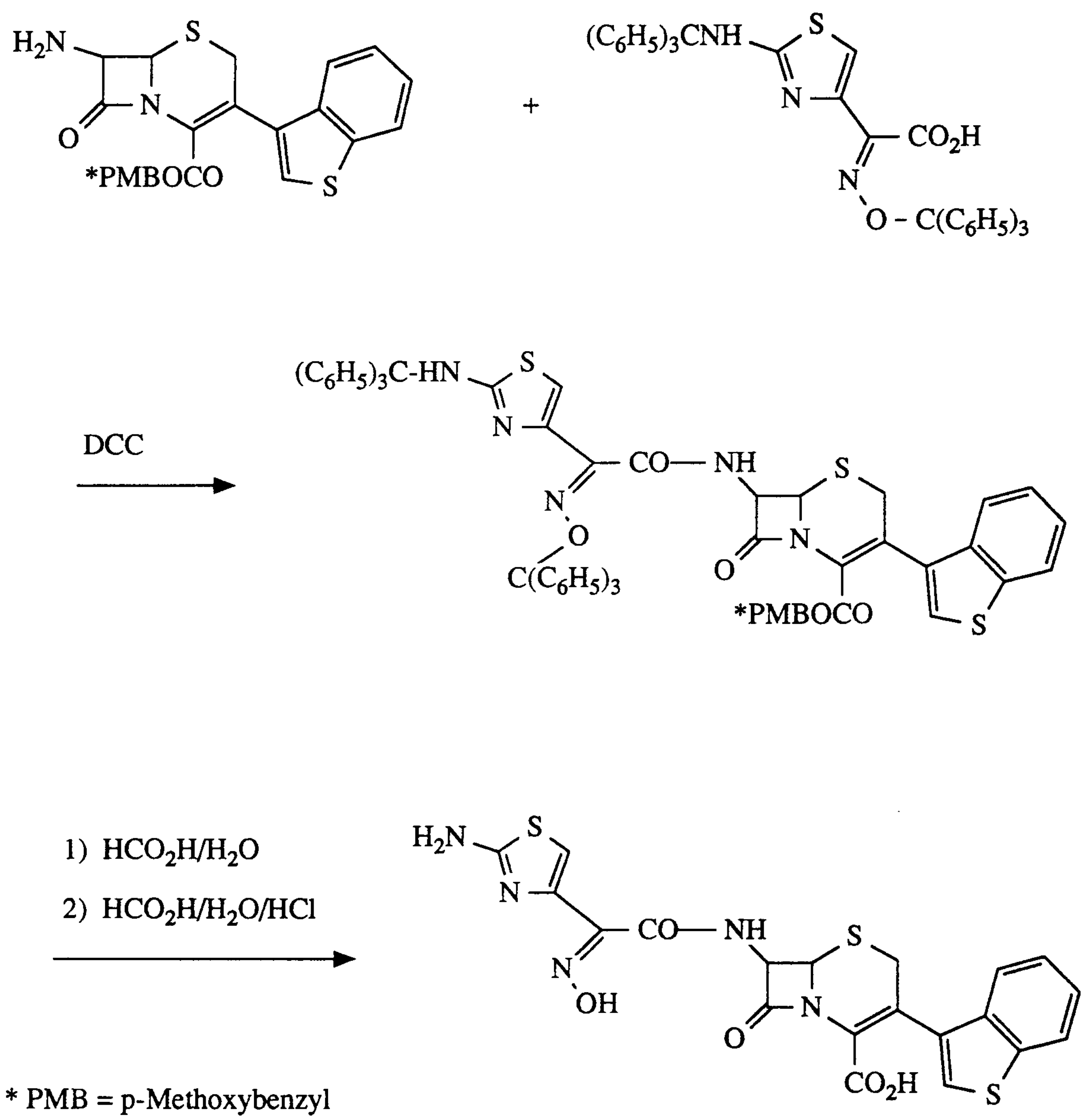


* PMB = p-Methoxybenzyl

Als Lösemittel eignen sich alle Solventien, die sich unter den Reaktionsbedingungen nicht verändern. Hierzu gehören bevorzugt Ether wie beispielsweise Diethylether, Dioxan oder Tetrahydrofuran, oder Chlorkohlenwasserstoffe wie Methylenchlorid, Chloroform oder Tetrachlormethan, oder Amide wie Dimethylformamid oder Hexamethylphosphorsäuretriamid, oder Acetonitril oder Aceton. Ebenso ist es möglich, Gemische der genannten Lösemittel einzusetzen. Bevorzugt ist Methylenchlorid.

Die Kondensation erfolgt im allgemeinen in einem Temperaturbereich von 0 ° C bis +60 ° C, vorzugsweise bei Raumtemperatur bei Normaldruck.

Die Aktivierung der Carboxylgruppe in den Verbindungen der allgemeinen Formel (III) erfolgt im allgemeinen durch Überführen in ein gemischtes Anhydrid mit Chlorameisensäureestern oder Sulfonsäurederivaten, wie beispielsweise Chlorameisensäureethylester, Chlorameisensäureisobutylester oder Methansulfonsäurechlorid, durch Überführen in das entsprechende Säurehalogenid, oder durch Überführen in einen aktivierten Ester, beispielsweise mit N-Hydroxybenztriazol oder Dicyclohexylcarbodiimid. Bevorzugt ist die Umsetzung mit Chlorameisensäureethylester und Methansulfonsäurechlorid.

Die Aktivierung der Aminfunktion der Verbindungen der allgemeinen Formel (II) erfolgt nach üblicher Methode, beispielsweise durch Überführung in die entsprechenden Silylderivate durch Umsetzung mit Bis-(trimethylsilyl)acetamid, N-trimethylsilylacetamid oder Bis(trimethylsilyl)harnstoff.

Als Hilfsstoffe werden bevorzugt Kondensationsmittel eingesetzt, die auch Basen sein können, insbesondere wenn beispielsweise die Carboxylgruppe als Anhydrid aktiviert vorliegt. Bevorzugt werden hier die

üblichen Kondensationsmittel wie Carbodiimide z.B. N,N'-Diethyl-, N,N'-Dipropyl-, N,N'-Diisopropyl-, N,N'-Dicyclohexylcarbodiimid, N-(3-Dimethylaminoisopropyl)-N'-ethylcarbodiimidhydrochlorid, oder Carbonylverbindungen wie Carbonyldiimidazol, oder 1,2-Oxazoliumverbindungen wie 2-Ethyl-5-phenyl-1,2-oxazolium-3-sulfonat oder 2-tert.-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Benzotriazolyloxy-tris(dimethylamino)phosphonium-hexafluorophosphat, oder als Basen Alkalicarbonate z.B. Natrium- oder Kaliumcarbonat oder -hydrogencarbonat, oder organische Basen wie Trialzylamine z.B. Triethylamin, N-Ethylmorpholin oder N-Methylpiperidin eingesetzt.

Die Abspaltung der Schutzgruppen erfolgt nach üblicher Methode, im Fall der Oxime gegebenenfalls sukzessive, entweder mit organischen Säuren, wie beispielsweise Ameisensäure, gegebenenfalls in Anwesenheit von Wasser oder in Anwesenheit von Wasser und einer Protonensäure, wie beispielsweise Salzsäure, bevorzugt mit Ameisensäure und Wasser oder Ameisensäure, Wasser und Salzsäure.

Im Fall, daß $R^3 \neq H$, ist es außerdem möglich, die Aminschutzgrupppe ($R^{13}$), die Carboxyschutzgruppen ($R^{2'}$) und die Schutzgruppen $R^{3'}$ und $R^{4'}$, gegebenenfalls in einem Schritt, mit Trifluoressigsäure abzuspalten.

Die Abspaltung der Schutzgruppen wird im allgemeinen in einem Temperaturbereich von 0 ° C bis +80 ° C, vorzugsweise bei Raumtemperatur durchgeführt.

Die Abspaltungen können sowohl bei Normaldruck, als auch bei erhöhtem oder erniedrigtem Druck (z.B. 0,5 bis 5 bar), vorzugsweise bei Normaldruck, durchgeführt werden.

Die Verbindungen der allgemeinen Formel (II) sind neu, können aber in Analogie zu literaturbekannten Verfahren [vgl. US 48 554 18; Tetrahedron Lett., Vol. 29, Nr. 47, 6043-6046, 1988] hergestellt werden, indem man beispielsweise 7-Phenacetylamino-3-trifluormethansulfonyl-3-cephem-4-carbonsäure-p-methoxybenzylester der Formel (V),

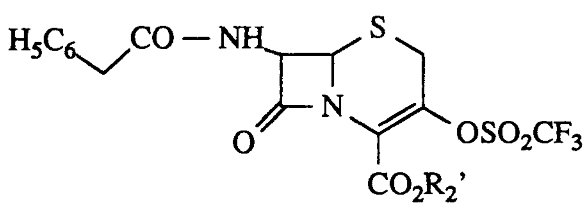

(V)

in welcher

$R^{2'}$ die oben angegebene Bedeutung hat,

mit Verbindungen der allgemeinen Formel (VI),

$R^1$-Z (VI)

in welcher

$R^1$ die oben angegebene Bedeutung hat

und

Z für einen im folgenden definierten Zinn-organyl-Rest steht,

in aprotisch, polaren Lösemitteln, in Anwesenheit von Metallhalogeniden, Phosphinen und einem Katalysator zu Verbindungen der allgemeinen Formel (VII),

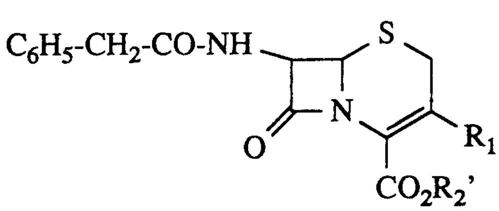

(VII)

in welcher

$R^1$ und $R^{2'}$ die oben angegebene Bedeutung haben,

umsetzt

und in einem letzten Schritt die Aminfunktion nach üblicher Methode, beispielsweise durch Einwirkung der Systems 1.) $PCl_5$/Pyridin/Methylenchlorid und 2.) $HN(C_2H_5)_2$/Methanol deblockiert.

Als Zinnorganyle (Z) eignen sich beispielsweise Trimethylstannyl, Triethylstannyl oder Tributylstannyl. Bevorzugt ist Tri-n-butylstannyl.

Als Metallhalogenide eignen sich Zink-, Lithium- und Magnesiumhalogenide, wie beispielsweise $ZnCl_2$, $ZnBr_2$, LiCl, LiBr, $MgCl_2$ oder $MgBr_2$. Bevorzugt ist $ZnCl_2$.

Als Katalysatoren eignen sich Pd(O)- und (II)-Komplexe, wie beispielsweise Bis(dibenzyliden-acetonyl)-palladium [$(Pd/dba)_2$], $Pd_2dba_3$ x $CHCl_3$ oder $Pd(OAc)_2$.Bevorzugt ist $Pd_2dba_3$ x $CHCl_3$.

Als aprotische, polare Losemittel eignen sich beispielsweise Acetonitril, Dimethylsulfoxid (DMSO), Dimethylformamid (DMF), oder Ether wie Glyme, Dioxan oder THF, oder Aceton, Hexamethylphosphoramid, oder N-Methylpyrrolidon oder 1-Methyl-2-pyrrolidon. Bevorzugt ist N-Methylpyrrolidon.

Als Phosphine können beispielsweise Triphenylphosphin, Tri-(3-fluorphenyl)-phosphin, Diphenylmethylphosphin, Tributylphosphin, Tri-(2-thienyl)-phosphin oder Tri-(2-furyl)phosphin eingesetzt werden. Bevorzugt ist Tri-(2-furyl)phosphin.

Die Reaktion wird in einem Temperaturbereich von -30 °C bis +90 °C, vorzugsweise bei Raumtemperatur und Normaldruck durchgeführt.

Die Verbindung der allgemeinen Formel (V) ist an sich bekannt [J. Org. Chem. 1989, 54, 4962-4966].

Die Verbindungen der allgemeinen Formel (VI) sind teilweise bekannt, können aber nach literaturbekannten Verfahren hergestellt und eingesetzt werden [vgl. EP 343 277 A1].

Die Verbindungen der allgemeinen Formel (VII) sind unter Berücksichtigung der oben angegebenen Definition für $R^1$ neu, können aber in Analogie zu bekannten Verfahren hergestellt werden [vgl. J. Org. Chem., Vol. 54, Nr. 20, 1989].

Die Verbindungen der allgemeinen Formel (III) sind an sich bekannt oder können nach üblicher Methode hergestellt werden [vgl. Tetrahedron, Vol. 34, 2233-2243].

Die Verbindungen der allgemeinen Formel (IV) sind neu und können nach dem oben angegebenen Verfahren hergestellt werden.

Die erfindungsgemäßen Verbindungen der allgemeinen Formel (I) weisen bei geringer Toxizität ein breites antibakterielles Spektrum gegen gram-positive und gram-negative Keime auf. Diese Eigenschaften ermöglichen ihre Verwendung als chemotherapeutische Wirkstoffe in der Human- und Tiermedizin.

Die erfindungsgemäßen Verbindungen sind gegen ein sehr breites Spektrum von Mikroorganismen wirksam. Mit ihrer Hilfe können gram-negative und gram-positive Bakterien und bakterienähnliche Mikroorganismen bekämpft sowie die durch diese Erreger hervorgerufenen Erkrankungen verhindert, gebessert und/oder geheilt werden.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch solche Erreger hervorgerufen werden.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Der oder die Wirkstoffe können gegebenenfalls in einem oder mehreren der oben angegebenen Trägerstoffe auch in mikroverkapselter Form vorliegen.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten. Im allgemeinen hat es sich sowohl in der Human- als auch in der Veterinärmedizin

als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30 mg/kg Körpergewicht.

Die neuen Verbindungen können in den üblichen Konzentrationen und Zubereitungen zusammen mit dem Futter bzw. Lactamaseinhibitoren z.B. mit Penicillinen, die besonders penicillinasefest sind und Clavulansäure kombiniert werden. Eine solche Kombination wäre z.B. die mit Oxacillin oder Dicloxacillin.

Die erfindungsgemäßen Verbindungen können zum Zweck der Erweiterung des Wirkungsspektrums und um eine Wirkungssteigerung zu erreichen auch mit Aminoglykosidantibiotika, wie z.B. Gentamicin, Sisomicin, Kanamycin, Amikacin oder Tobramycin kombiniert werden.

Zu allen Verbindungen liegen $^1$H-NMR-Daten vor.

Ausgangsverbindungen

Beispiel I

3-Benzo[b]thienyl-tri-n-butyl-stannan

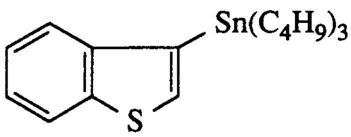

Zu einer Lösung von 150 mmol n-Butyllithium in 500 ml wasserfreiem THF tropft man bei -78 °C 32,0 g (150 mmol) 3-Brom-benzo[b]thiophen. Man rührt 1 h bei -78 °C, gibt 52,1 g (160 mmol) Tri-n-butylzinnchlorid zu und läßt auf Raumtemperatur erwärmen. Zur Aufarbeitung wird gesättigte Ammoniumchloridlösung zugegeben und mit Ether extrahiert. Nach Chromatographie an Kieselgel mit Petrolether erhält man 14,8 g (23% der Theorie) der Titelverbindung.

Beispiel II

3-(3-Benzo[b]thienyl)-7β-phenacetylamino-3-cephem-4-carbonsäure-p-methoxybenzylester

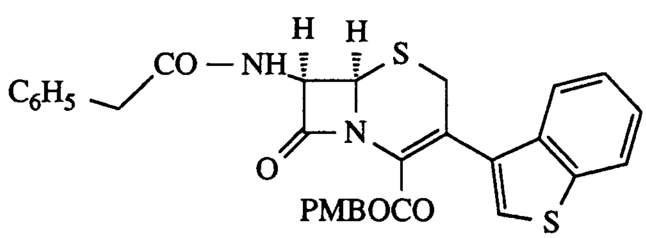

Man läßt 4,7 g (8,0 mmol) 7β-Phenacetylamino-3-trifluormethan-sulfonyloxy-3-cephem-4-carbonsäure-p-methoxybenzylester mit 3,4 g (8,0 mmol) der Verbindung aus Beispiel I, 1,9 g (13,9 mmol) wasserfreiem Zinkchlorid, 0,34 g Tri-2-furylphosphin und 0.35 g (0.34 mmol) Tris-(dibenzylidenaceton)-dipalladium-chloroform in 130 ml wasserfreiem N-Methylpyrrolidon unter Argon bei 50 °C über Nacht reagieren. Anschließend wird auf 400 ml Wasser gegossen und der ausgefallene Niederschlag wird abgesaugt. Der Rückstand, gelöst in 400 ml Methylenchlorid, wird mit Natriumsulfat getrocknet und nach dem Entfernen des Lösemittels an Kieselgel mit Toluol/Essigester (9:1) chromatographiert.
Ausbeute: 3,2 g (70% der Theorie).

Beispiel III

7β-Amino-3-(3-benzo[b]thienyl)-3-cephem-4-carbonsäure-p-methoxybenzylester

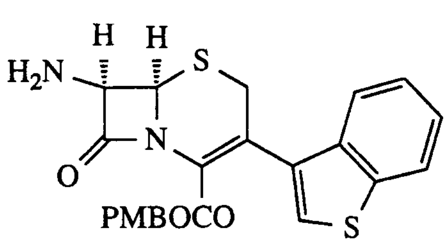

Zu einer Lösung von 4,4 g (7,7 mmol) der Verbindung aus Beispiel II und 1,6 ml (19,8 mmol) Pyridin in 80 ml wasserfreiem Methylenchlorid gibt man bei -20 ° C 3,3 g (15,8 mmol) Phosphorpentachlorid. Man rührt 5 min bei -20 ° C, 10 min bei 0 ° C, 1 h bei 15 ° C und kühlt anschließend auf -78 ° C ab. 80 ml auf -78 ° C gekühltes Methanol werden schnell zugegeben. Die lösung wird 5 min bei -78 ° C, 10 min bei 0 ° C und 25 min bei 15 ° C gerührt. Nach dem Abkühlen auf -15 ° C gibt man 0,95 ml (9,2 mmol) Diethylamin zu und hält 10 min bei dieser Temperatur. Zur Aufarbeitung gießt man auf 150 ml gesättigte Natriumhydrogencarbonat-Lösung und extrahiert mit Methylenchlorid. Die vereinigten organischen Phasen werden mit Natriumhydrogencarbonat-Lösung und Wasser gewaschen, mit Natriumsulfat getrocknet und eingeengt. Der Rückstand wird an Kieselgel mit Toluol/Essigester (3:1) chromatographiert.
Ausbeute: 2,9 g (83% der Theorie)

Beispiel IV

3-(3-Benzo[b]thienyl)-7β-[2-(2-tritylamino-4-thiazolyl)-2 syn-trityloxyimino-acetylamino]-3-cephem-4-carbonsäure-p-methoxybenzylester

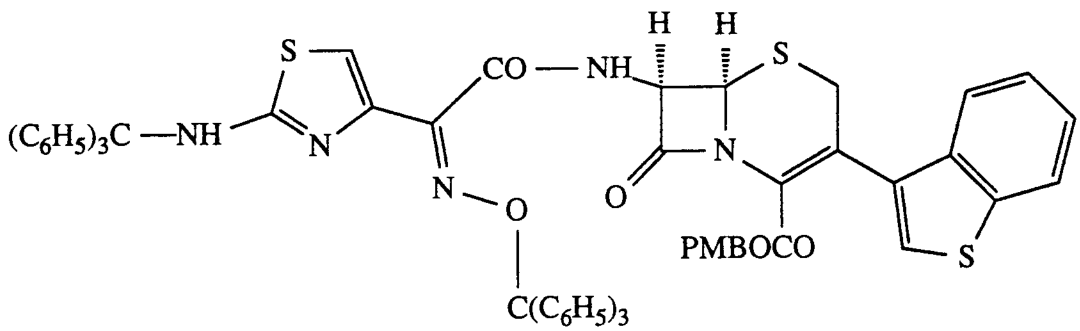

Eine Lösung von 6,9 g (10 mmol) 2-(2-Tritylamino-4-thiazolyl)-2-syn-trityloxyimino-essigsäure in 100 ml Methylenchlorid wird mit 1,4 g (7 mmol) Dicyclohexylcarbodiimid 2 h bei Raumtemperatur gerührt. Man fügt 1,7 g (4 mmol) der Verbindung aus Beispiel III zu und rührt über Nacht bei Raumtemperatur. Nach dem Abdestillieren des Lösemittels wird an Kieselgel mit Toluol chromatographiert.
Ausbeute: 2,8 g (64% der Theorie)

Beispiel V

3-(3-Benzo[b]thienyl)-7β-[2-(2-t-butyloxycarbonylamino-4-thiazolyl)-2-syn-methoxyimino-acetylamino]-3-cephem-4-carbonsäure-p-methoxybenzylester

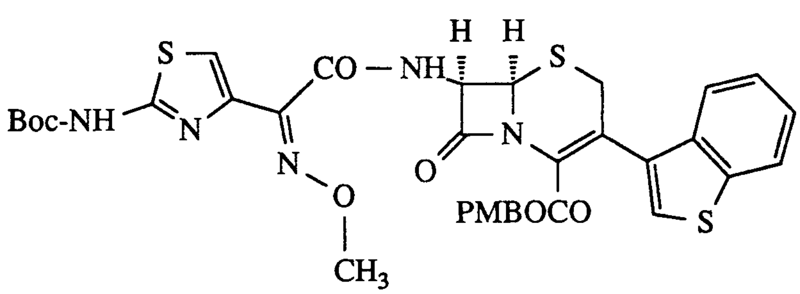

Eine Lösung von 0,6 g (1,3 mmol) der Verbindung aus Beisipel III, 1,2 g (4 mmol) 2-(2-t-Butyloxycarbonylamino-4-thiazolyl)-2-syn-methoxyiminoessigsäure und 0,85 g (4 mmol) Dicyclohexylcarbodiimid in 40 ml Acetonitril wird über Nacht gerührt. Man engt ein und chromatographiert an Kieselgel mit Toluol/Essigester (5:1).
Ausbeute: 0.8 g (85% der Theorie)

Herstellungsbeispiele

Beispiel 1

7β-[2-(2-Amino-4-thiazolyl)-2-syn-hydroxyimino-acetylamino]-3-(3-benzo[b]thienyl)-3-cephem-4-carbonsäure-p-methoxybenzylester

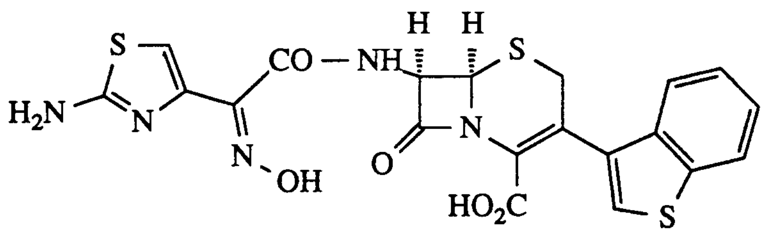

2,8 g (2,5 mmol) der Verbindung aus Beispiel IV werden mit 20 ml 90%iger Ameisensäure 1 h bei Raumtemperatur gerührt. Anschließend gibt man 1 ml konz. Salzsäure zu und rührt weitere 2 h bei Raumtemperatur. Man engt im Vakuum zur Trockene ein und chromatogrpahiert an HP-20 mit Wasser/Acetonitril.
Ausbeute: 1,0g (80% der Theorie)
$^1$H-NMR (DCOOD): 3,98 (s, 2H, 2-H); 5,46 (d, J = 5 Hz, 1H, 6-H); 6,13 (d, J = 5 Hz, 1H, 7-H); 7,42 (s, 1H, 7'-Ar-H); 7,35 - 7,45 (m, 3H, 3'-Ar-H), 7,78 - 7,89 (m, 2H, 3'-Ar-H).

Beispiel 2

7β-[2-(2-Amino-4-thiazolyl)-2-syn-methoxyimino-acetylamino]-3-(3-benzo[b]thienyl-3-cephem-4-carbonsäure-p-methoxybenzylester(Trifluoracetat)

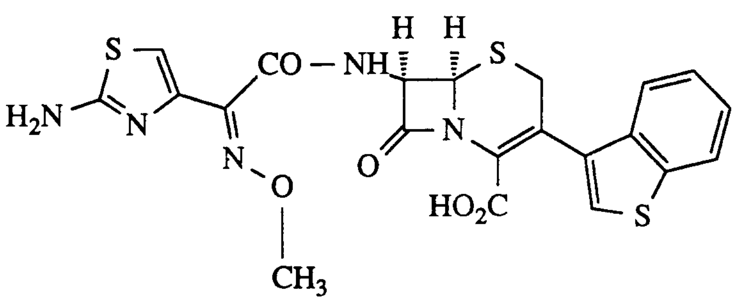

x $CF_3CO_2H$

0,8 g (1,1 mmol) der Verbindung aus Beispiel V werden mit einer Mischung aus 1 ml Anisol und 50 ml Trifluoressigsäure 1 h bei Raumtemperatur behandelt. Anschließend wird die Trifluoressigsäure im Vakuum abgezogen und der Rückstand mit Diisopropylether verrührt.
Ausbeute: 0,6 g (87% der Theorie)
$^1$H-NMR (DCOOD): 4,00 (s, 2H, 2-H); 4,17 (s, 3H, $OCH_3$); 5,44 (d, J = 5 Hz, 1H, 6-H); 6,12 (d, J = 5 Hz, 1H, 7-H); 7,37 (s, 1H; 7'-Ar-H); 7,40 - 7,45 (m, 3H, 3'-Ar-H); 7,80 -7.90 (m, 2H, 3'-Ar-H).

**Patentansprüche**

**1.** Verbindungen der allgemeinen Formel (I),

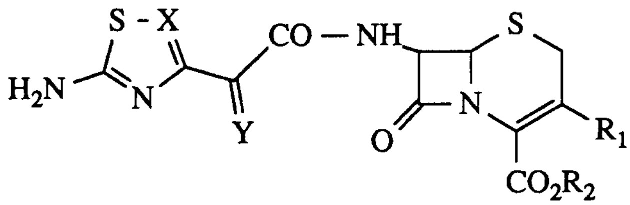

(I)

in welcher

X für ein Stickstoffatom oder für die -CH-Gruppe steht,

Y für eine Gruppe der Formel N-$OR^3$ oder $CHR^4$ steht,
worin

$R^3$ Wasserstoff, geradkettiges oder verzweigtes Alkenyl, Alkinyl oder Alkyl mit jeweils bis zu 8 Kohlenstoffatomen bedeutet, wobei letzteres gegebenenfalls durch Halogen oder durch geschütztes oder ungeschütztes Carboxy oder Amino substituiert sein kann,

$R^4$ Wasserstoff, Aryl mit 6 bis 10 Kohlenstoffatomen, geschütztes oder ungeschütztes Carboxy, Halogen oder geradkettiges oder verzweigtes Alkoxycarbonyl, Alkoxy, Alkenyl oder Alkyl mit jeweils bis zu 8 Kohlenstoffatomen bedeutet, wobei letzteres gegebenenfalls durch Halogen, Hydroxy, Nitro, Cyano, Carboxy oder durch geradkettiges oder verzweigtes Alkoxy oder Alkoxycarbonyl mit jeweils bis zu 6 Kohlenstoffatomen substituiert sein kann,

$R^1$ für einen Rest der Formel

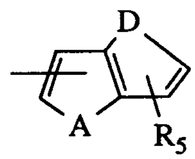

oder

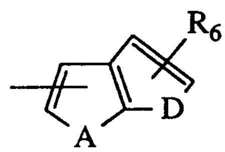

steht,
worin

A und D gleich oder verschieden sind und die Gruppe -CH=CH oder ein Sauerstoff- oder Schwefelatom bedeuten
und

$R^5$ und $R^6$ gleich oder verschieden sind und Halogen, Trifluormethyl, Trifluormethoxy, Hydroxy oder eine Gruppe der Formel -$NR^7R^8$ bedeuten,
worin

$R^7$ oder $R^8$ gleich oder verschieden sind und Wasserstoff, geradkettiges oder verzweigtes Alkyl mit bis zu 8 Kohlenstoffatomen oder Phenyl bedeuten,
oder geradkettiges oder verzweigtes Alkoxy oder Alkyl mit jeweils bis zu 8 Kohlenstoffatomen bedeuten, wobei letzteres durch Hydroxy, Halogen oder durch geradkettiges oder verzweigtes Alkoxy mit bis zu 6 Kohlenstoffatomen substituiert sein kann,

$R^2$ für Wasserstoff, oder
für eine in der $\beta$-Lactamchemie übliche Carboxyschutzgruppe oder für einen in vivo spaltbaren Esterrest steht

und deren pharmazeutisch verträglichen Salze.

**2.** Verbindungen gemäß Anspruch 1, in denen

$R^2$ steht für:
eine in vivo abspaltbare Estergruppe der folgenden Formeln:

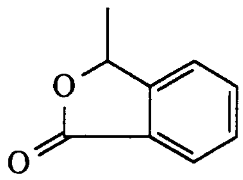, 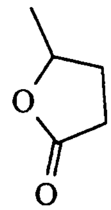, 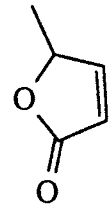, $-CH(CO_2C_2H_5)-OC_2H_5$

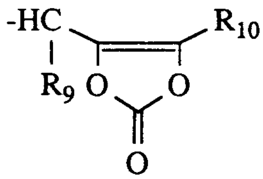, 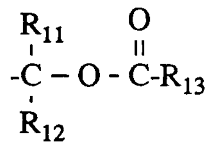 oder 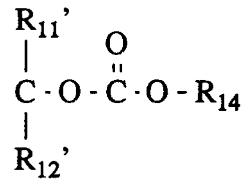

worin

| | |
|---|---|
| $R^9$ und $R^{10}$ | gleich oder verschieden sind und Wasserstoff, Phenyl oder geradkettiges oder verzweigtes Alkyl mit bis zu 4 Kohlenstoffatomen bedeuten, |
| $R^{11}$, $R^{11'}$, $R^{12}$ und $R^{12'}$ | gleich oder verschieden sind und Wasserstoff oder geradkettiges oder verzweigtes Alkyl mit bis zu 4 Kohlenstoffatomen bedeuten, |
| $R^{13}$ | geradkettiges oder verzweigtes Alkyl mit bis zu 6 Kohlenstoffatomen bedeutet, |
| $R^{14}$ | geradkettiges oder verzweigtes Alkyl mit bis zu 6 Kohlenstoffatomen oder Cyclohexyl bedeutet. |

**3.** Verbindungen der allgemeinen Formel (I),

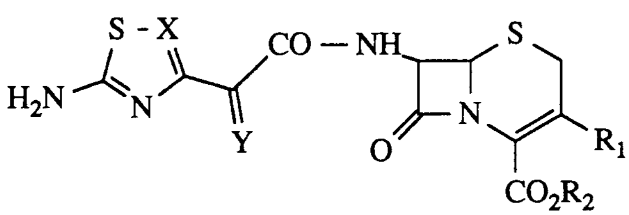

(I)

in welcher

| | |
|---|---|
| X | für ein Stickstoffatom oder für die -CH-Gruppe steht, |
| Y | für eine Gruppe der Formel $N-OR^3$ oder $CHR^4$ steht, worin |
| $R^3$ | Wasserstoff oder geradkettiges oder verzweigtes Alkyl mit bis zu 6 Kohlenstoffatomen bedeutet, das gegebenenfalls durch Fluor, Chlor, Brom oder geschütztes oder ungeschütztes Carboxy oder Amino substituiert ist, |
| $R^4$ | Wasserstoff, Phenyl, Carboxy, Fluor, Chlor, Brom oder geradkettiges oder verzweigtes Alkoxy, Alkoxycarbonyl oder Alkyl mit jeweils bis zu 6 Kohlenstoffatomen bedeutet, wobei letzteres durch Fluor, Chlor, Brom, Hydroxy, Carboxy oder durch geradkettiges oder verzweigtes Alkoxy oder Alkoxycarbonyl mit jeweils bis zu 4 Kohlenstoffatomen substituiert sein kann, |
| $R^1$ | für einen Rest der Formel |

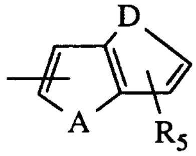 oder 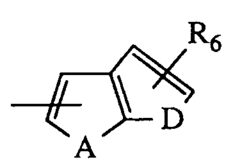

steht
worin

A ein Schwefelatom bedeutet
und

D die -CH = CH-Gruppe oder ein Schwefelatom bedeutet,

$R^5$ und $R^6$ gleich oder verschieden sind und Fluor, Chlor, Brom, Amino oder geradkettiges oder verzweigtes Alkoxy oder Alkyl mit jeweils bis zu 4 Kohlenstoffatomen bedeuten, wobei letzteres durch Hydroxy, Fluor, Chlor, Methoxy oder Ethoxy substituiert sein kann,

$R^2$ für Wasserstoff steht, oder
für einen Rest der Formel

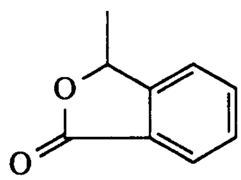 ,

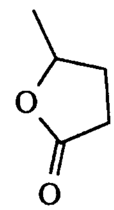 ,

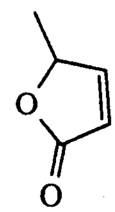

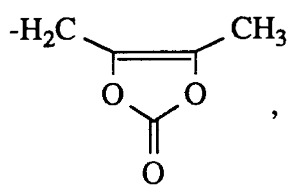 ,

$-CH_2-OCO-C(CH_3)_3$,
$-CH(CH_3)-OCOOC_2H_5$ ,
$-CH_2-OCOCH_3$ ,
$-CH-(CH_3)-O-CO-CH_3$ ,
$-CH(CH_3)-O-COO-CH_3$ , $-CH(CH_3)-O-COO-CH(CH_3)_2$ ,
$-CH(CH_3)-O-COO-C_6H_{11}$ oder $-CH(CO_2C_2H_5)-OC_2H_5$ steht

und deren pharmazeutische verträgliche Salze.

**4.** Verbindungen der allgemeinen Formel (I),

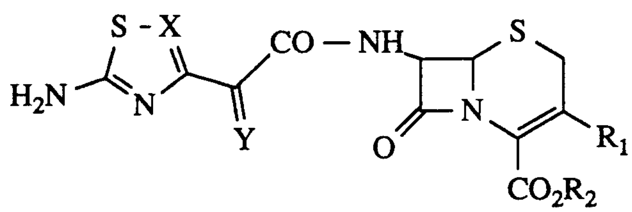

(I)

in welcher

X für ein Stickstoffatom oder für die -CH-Gruppe steht,

Y für eine Gruppe der Formel $N-OR^3$ oder $-CHR^4$ steht,
worin

$R^3$ Wasserstoff oder geradkettiges oder verzweigtes Alkyl mit bis zu 4 Kohlenstoffatomen bedeutet, das gegebenenfalls durch Fluor, Chlor oder durch geschütztes oder ungeschütztes Carboxy oder Amino substituiert ist,

$R^4$ Wasserstoff oder geradkettiges oder verzweigtes Alkoxy oder Alkyl mit jeweils bis zu 4 Kohlenstoffatomen bedeutet, wobei letzteres durch Hydroxy, Carboxy, Methoxy, Ethoxy oder Propoxy substituiert sein kann,

$R^1$ für einen Rest der Formel

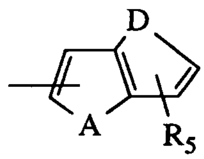 oder 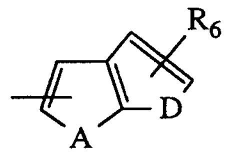

steht
worin

A ein Schwefelatom bedeutet
und

D die -CH = CH-Gruppe oder ein Schwefelatom bedeutet,

$R^5$ und $R^6$ gleich oder verschieden sind und Fluor, Chlor, Brom, Amino oder geradkettiges oder verzweigtes Alkoxy oder Alkyl mit jeweils bis zu 4 Kohlenstoffatomen bedeuten, wobei letzteres durch Hydroxy, Fluor, Chlor, Methoxy oder Ethoxy substituiert sein kann,

$R^2$ für Wasserstoff steht, oder für einen Rest der Formel

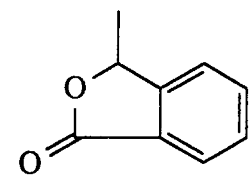 , 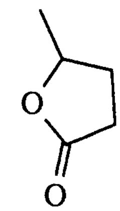 , 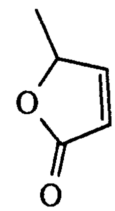

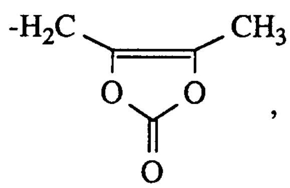 ,

$-CH_2-OCO-C(CH_3)_3$ ,
$-CH(CH_3)-OCOOC_2H_5$ ,
$-CH_2-OCOCH_3$ ,
$-CH-(CH_3)-O-CO-CH_3$ ,
$-CH(CH_3)-O-COO-CH_3$ , $-CH(CH_3)-O-COO-CH(CH_3)_2$ ,
$-CH(CH_3)-O-COO-C_6H_{11}$ oder $-CH(CO_2C_2H_5)-OC_2H_5$ steht

und deren pharmazeutische verträgliche Salze. Ganz besonders bevorzugt sind die Natriumsalze und die Hydrochloride.

**5.** Verfahren zur Herstellung der Verbindungen der allgemeinen Formel (I),

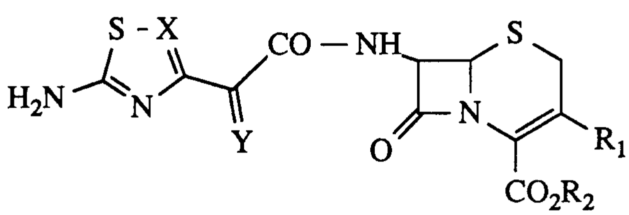 (I)

dadurch gekennzeichnet, daß man Verbindungen der allgemeinen Formel (II),

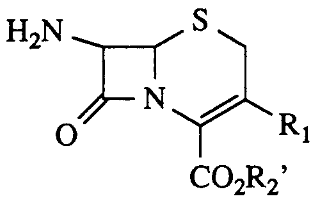

(II)

in welcher

$R^1$ die oben angegebene Bedeutung hat

und

$R^{2'}$ für eine der oben angegebenen Carboxyschutzgruppen, insbesondere für p-Methoxybenzyl steht,

gegebenenfalls auch unter Aktivierung der Aminfunktion, mit Verbindungen der allgemeinen Formel (III),

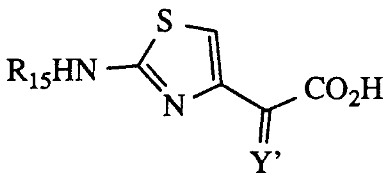

(III)

in welcher

Y' für eine der oben aufgeführten Gruppen N-$OR^{3'}$ oder $CHR^{4'}$ steht, worin

$R^{3'}$ die obenangegebene Bedeutung von $R^3$ hat oder für eine der oben aufgeführten Oximschutzgruppen, insbesondere für den Tritylrest steht,

$R^{4'}$ die oben angegebene Bedeutung von $R^4$ hat oder für geschütztes Carboxy steht und

$R^{15}$ ebenfalls für eine der oben aufgeführten Aminoschutzgruppen, vorzugsweise für Boc oder für den Tritylrest steht, in inerten Lösemitteln, gegebenenfalls unter Aktivierung der Carboxylgruppe in Anwesenheit eines Hilfsstoffes,

zunächst zu den Verbindungen der allgemeinen Formel (IV),

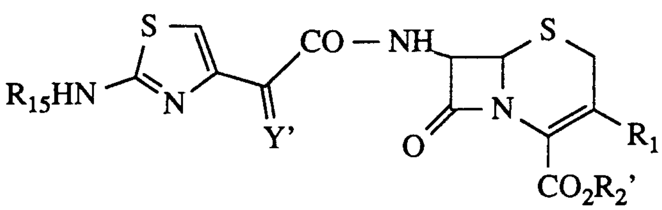

(IV)

in welcher

$R^1$, $R^{2'}$, $R^{15}$ und Y' die oben angegebene Bedeutung haben,

umsetzt und anschließend die Schutzgruppe $R^{2'}$, $R^{3'}$, $R^{4'}$ (Y') und $R^{15}$ nach üblicher Methode, gegebenenfalls in 2 Schritten abspaltet und die gewünschten Salze oder aus den Salzen die freien Säuren herstellt.

6. Verwendung der Verbindungen der allgemeinen Formel (I) gemäß den Ansprüchen 1 bis 4 zur Bekämpfung von Krankheiten des menschlichen und tierischen Körpers.

7. Verwendung der Verbindungen der allgemeinen Formel (I) gemäß den Ansprüchen 1 bis 4 zur Herstellung von Arzneimitteln.

8. Tierfuttermittel bzw. Tierfutterprämixe, gekennzeichnet durch einen Gehalt an Cephalosporinen gemäß den Ansprüchen 1 bis 4.